# EUROPEAN PATENT SPECIFICATION

(11) **EP 0 524 301 B1**
(45) Date of publication and mention of the grant of the patent: **24.07.2002**
(21) Application number: 92906299.0
(22) Date of filing: 11.02.1992
(51) Int. Cl.: G01N 21/41, G01N 33/543, G01N 33/544, G01N 33/545, G01N 33/551, G01N 33/552, G01N 33/55

(54) **ELLIPSOMETRIC IMMUNOASSAY SYSTEM AND METHOD INCLUDING A THIN FILM DETECTION DEVICE**
ELLIPSOMETRISCHES IMMUNTESTSYSTEM UND -METHODE MIT EINER DÜNNSCHICHT-NACHWEISVORRICHTUNG
SYSTEME ET PROCEDE D'ANALYSE IMMUNOLOGIQUE ELLLIPSOMETRIQUE COMPORTANT UN DISPOSITIF DE DETECTION A COUCHES MINCES

(30) Priority: 11.02.1991 US 653064
(43) Date of publication of application: 27.01.1993
(60) Divisional application: 01111726.4
(73) Proprietor: Thermo BioStar, Inc., Boulder, CO 80301 (US)
(72) Inventor: ETTER, Jeffrey, Boulder, CO 80302 (US); MAUL, Diana, Thornton, CO 80221 (US); BOGART, Greg, Fort Collins, CO 80524 (US); ZAPP, Loretta, Boulder, CO 80303 (US); PETERSON, Tammy, Broomfield, CO 80020 (US)
(74) Representative: Sexton, Jane Helen
(86) International application number: US9200809
(87) International publication number: WO9214136

(56) References cited:
- EP-A- 0 197 266
- WO-A-89/09937
- US-A- 4 357 142
- US-A- 4 508 832
- US-A- 4 992 385
- RESEARCH DISCLOSURE vol. 309, no. 088, 10 January 1990, HAVANT GB ANONYMOUS 'Improvement in the wet-adhesion of polymers to Si-wafers'

## Description

### FIELD OF INVENTION

Thin film detection systems, whether visually interpretable or instrumentally examined, for use in quantifying interacting species reaction of biological significance have long suffered due to the lack of sufficiently dense and reactive receptive materials on the test surface.

This invention is concerned with the production of a test surface for thin film detection systems. The invention is further concerned with a method for studying the surface properties on the test surface. Specifically, the invention pertains to the production of a test surface that permits a wide variety of receptive material to be secured to the test surface such that various tests for detection of different analytes can be inexpensively produced. The test surface used in combination with optical detection forms a highly sensitive system for the detection of a specific analyte.

This invention is concerned with a specifically reactive surface prepared in the form of a thin chemical film(s) of limited surface area immobilized on a solid phase substrate that constitutes or forms the surface layer of a slide, wafer, or other platform or object, whether flat or cylindrical, continuous, or of any other shape or structure. The specific reaction layer may serve a variety of functions, including, but not limited to detection or measurement, as in air or water pollution monitoring, immunoassays or other solid phase events involving interacting species and employs optical direct thin film monitoring methods.

The invention is further concerned with a method of constructing layers of materials which mediately bind the specific reaction layer to the substrate of a test surface for the purpose of optimizing the performance of the device. Specifically, the invention pertains to a method of utilizing an intermediate layer of assay performance optimizing material(s) that allows control of the density, stability, and viability of reactive material immobilized on a surface.

### BACKGROUND OF THE INVENTION

The ability to control various physical and chemical properties of thin films immobilized on solid surfaces has proven essential to industries as diverse as the semiconductor and diagnostics industries. In the former, for example, the thickness and homogeneity of thin layers of insulating, semiconducting, or conducting materials on silicon wafers must be controlled to optimize the performance of integrated circuits embedded in the wafers. In the diagnostic industry, the density and stability of immobilized receptive material (or, in some case, enzymes) must be controlled to optimize the performance of assay test surface or device.

While the control of certain thin film properties in development and production has made various products and devices and even entire industries possible, limitations in the control of these properties and the failure of established art to control other thin film properties have delayed the development and marketing of a number of important technologies. These technologies include, but are not limited to, solid phase assay systems that directly monitor, without the use of labels, the concentration-dependent quantities of analyte that have bound specifically to receptive material on a solid phase, whether for in vitro diagnosis, in vivo monitoring, or other purposes. These and other potential or emerging technologies often share severe and unprecedented limitations in the macroscopic and/or microscopic surface area of the thin reactive films they can employ and in their tolerance for nonspecific interactions with these films. The state-of-the-art in thin film engineering generally does not provide the appropriate immobilization and binding schemes to assure the sensitivity or reactivity and the specificity required by technologies such as those listed above.

A case in point is the difficulty of adapting direct thin film monitoring systems such as those employed in the semiconductor industry for the purposes of direct solid phase assays. A number of optical thin film monitoring technologies including, for example, ellipsometry, multiple angle reflectometry, interference spectroscopy, and various other forms or combinations of polarimetry, reflectometry, spectroscopy, and spectrophotometry have found broad application in the semiconductor and integrated circuit industries for research and development, product development, and for the quality control of the thickness and homogeneity of thin film layers produced on the surface of wafers composed of silicon or other materials. There has been a growing interest in the application of such technologies to the detection or measurement of changes in the thickness, density, or mass of thin films resulting from the concentration-dependent immobilization of analytes of interest on a surface of suitably selected receptive material. Such thin film assay technologies, because they would directly detect or quantify the material of interest, represent promising alternatives to conventional solid phase assays which too often sacrifice needed accuracy or convenience as a result of their dependence upon the indirect measurement or detection of radioactive, enzyme, fluorescent, or luminescent labels as qualitative or quantitative indicators of the concentration-dependent immobilization of the analyte of interest. Although the potential advantages of thin film assay technologies often are appreciated, thin film engineering problems of the kind indicated above have obstructed the development of test kits suitable for diagnostic or other analytical markets. One problem is associated with the extremely limited macroscopic and/or microscopic surface area of the test films generally employed in thin film assays as compared with the microscopically convoluted surfaces characteristic of conventional solid phase assays. In most cases, there is a need to coat the substrate evenly with a continuous intermediate layer of material that protects the receptive material from the toxic effects of the reflective substrate.

In conventional solid phase assays, the larger test surfaces generally employed, such as those associated with the coated inside surfaces of microtiter plates or tubes, combined with the larger microscopic surface areas available for ligand molecule immobilization, allow the sheer number of ligand molecules immobilized to compensate for any sparsity of ligand coverage, any loss of its viability, and any possible loss of orientation of the receptive material. In direct thin film assays, on the other hand, severe limitations in the effective testing surface area demand, if adequate sensitivity is to be achieved, the use or development of special materials and procedures designed to maximize or optimize the density, viability, accessibility, and stability of the receptive material.

Another problem of thin film assays arises in the special case of optical assays, such as those that employ ellipsometric, interferometric, light scattering, total internal reflection, or other reflectometric monitoring systems where there are optical limitations on acceptable substrates. When receptive material for use in an assay is attached directly to such optical substrates, the substrate can exhibit toxic or semi-toxic effects on the receptive material. This is to say that the substrate may alter the conformation or chemical structure of many ligand molecules diminishing or destroying their viability. Biological materials and other macromolecules often used as ligands are especially subject to such toxic effects. This is a serious problem in the light of the surface area limitations in the thin film test surfaces.

Much of the original work to adapt siliceous materials for retention of biological molecules focused on affinity chromatography and silica (SiO₂) gel and related solid supports such as glass.

Initial activation of silica towards the biomolecule was accomplished by treatment with a silane. Silanization, regardless of the process used to apply the silane, can introduce functional groups capable of attaching the biomolecule by chemical means. There are a wide variety of chemistries available for attachment of the biomolecule to the functional groups introduced by the silane. These techniques have also been widely used in the production of biosensors. One of the more commonly utilized silanes in the prior art is N-(2-Aminoethyl-(3-aminopropyl)-trialkoxy silane.

When silicon replaces silica as the solid support or substrate for subsequent bioattachment, conventional silanization processes are inadequate in providing the desired characteristics. A silane requires the presence of silanol residues in order to attach to the surface. When silanol density is insufficient to yield the level of functional groups necessary for immobilization reactions, insufficient receptive material will be attached to the test surface. Silica and many glasses inherently possess or may be treated to provide high silanol content. This is not the case for silicon. Silicon also introduces surface effects not observed with silica or glass which are toxic or detrimental to biomolecules. Conventional silanization processes also produce hazardous waste materials which require disposal and in many cases the processes are tedious and difficult to monitor and control.

A variety of methods have been introduced to eliminate the silanization and subsequent chemical processes from the production of bioassay test surfaces. For example, U.S. Patent 4,169,138 attempts to improve antibody functionality, i.e., reactive density by coating a support or substrate with a biopolymer which improves antibody density (mass) it clearly can improve assay performance. This technique is of very limited utility in the production of test surfaces.

Various spacing and/or linking strategies, including the use of avidin and biotin, are known to improve the accessibility of antibodyligands through the separation and/or site-specific binding of the antibodies relative to the surface (as reviewed, for example, in Journal of Immunological Methods, 71 (1984), 133-140; Journal of Immunoassay, 6, 1&2 (1985), 67-77; Journal of Immunological Methods, 82 (1985), 215-224; BioChromatography, 3,4 (1988), 156-160*.*

Coating of the solid substrate with a polymer has also been used for subsequent attachment of a ligand or receptive material (see For exemple EP 254575). The primary emphasis in this approach has been to produce a surface capable of covalently binding the biomolecule to plastic or glass. Representative prior art includes U.S. Patent 4,354,884, U.S. Patent 4,357,142, U.S. Patent 4,444,879, U.S. Patent 4,415,665, U.S. 4,623,629, U.S. Patent 4,724,207, or U.S. Patent 4,363,634. These polymers may be biological or synthetic origin. Representative materials include styrene-butadiene copolymer, photoactivable polymers, proteins, polysaccharides, and polyamides. U.S. Patent 4,591,550 and U.S. Patent 4,849,330 describe a coating of a doped silicon substrate with a lipid bilayer for subsequent covalent immobilization of a biomolecule in a electrochemical or impedance enzymatic based detection system.

None of these approaches has adequately addressed the production of test surfaces or surfaces which are compatible with the direct optical detection of an analyte. Direct optical detection would include optical interference effects (visually examined or measured by reflectance-photometry) ; thin film measurement methods such as ellipsometry, interferometry, adsorptive, or transmissive measurement.

Ideally, for direct optical detection methodologies the surface activation technique should provide a covalent modification of the surface for stability, while introducing a very dense, uniform or conformal film on the surface of the substrate. In certain instances a strongly adsorbed conformal film without covalent attachment may be adequate. Suitable substrates such as silicon; macroscopically planar, uniform optical glasses, metalized glass and plastic, or plastic whether or not coated with an optical layer (i.e., SiO, SiO₂, SiN, etc.) share a deficiency of available reactive groups for covalent attachment. Once applied the surface modification layer or intermediate layer should provide an environment which supports the covalent or adsorptive attachment of a biomolecule layer that is dense, functional, and stable. This surface modification layer or barrier, designated as the intermediate layer, must be of sufficient thickness or density to insulate the biomolecule layer from the toxic effects of the substrate. The ligand or receptive material is defined as one member of a group of interacting species which could include, but is not limited to, antigen/antibody, enzyme/substrate, oligonucleotide/DNA, chelator/metal, enzyme/inhibitor, bacteria/receptor, virus/receptor, hormone/receptor, DNA/RNA, or RNA/RNA and binding of any of these species to another species as well as the interaction of other non-biological species.

Non-linear branched polymeric siloxanes meet the requirements of covalent attachment to the substrate and will adhere receptive materials in a dense, reactive, stable film. These polymers typically contain 2-3 branch points which may provide a number of different functionalities; including aminoalkyl, carboxypropyl, chloropropyl, mercaptopropyl, phenethyl, phenethylsulfonate, vinyl, and methacryloxypropyl. Typical applications of these materials include: treatment of textiles for water repellency; internal mold release agents for nylon production; leather finishes; detergent resistant polish formulations; contact lens production (U.S. Patent 4,208,506); polyurethane foam production formulations (U.S. Patent 3,530,159); and for release coatings for utensils (U.S. Patent 4,369,268). A combination of silanes could also be used to create branch points on a linear silane. However, such a multiple step process is less attractive for manufacturing. Although it is conceivable that silanes could be used to generate branch points in a single step process. These materials may be utilized for covalent or passive adherence of the receptive material.

### SUMMARY OF THE INVENTION

One of the objects of this invention is to provide a versatile test surface for use in a direct thin film detection system.

Another object of this invention is to provide a test surface that can be formed of light interacting material while providing a favorable environment for the binding of receptive material.

A further object of this invention is to provide a test surface that can be fabricated easily and efficiently with a dense, viable layer of receptive material.

Another object of the present invention is to permit the receptive material to be passively adsorbed or covalently attached to the test surface by a variety of immobilization chemistries.

Another object of the present invention is making easier and more cost efficient the production of test surfaces.

An object of the present invention is to make a test surface with increased sensitivity to low levels of analytes that can be optically determined.

Still a further object of the present invention is to improve the functionality and stability of the receptive material.

Yet another object of the present invention is to teach a general strategy for the mediated binding of receptive material to a test surface for use in an interactive species assay which employs optical thin film detection or measurement technology.

The present invention provides a thin film device as defined in claim 1.

The substrate can consist of a planar reflective material and with the attached layer can polarize radiation ellipsometrically upon reflection.

It will be understood the described assay device can be used in an ellipsometric immunoassay system comprising an ellipsometer and said device.

The present invention also provides a method of producing this optical device as defined in claim 1 wherein a substrate is provided, the intermediate layer is applied by a spin coating technique, a spray coating technique or a dip coating technique, and the receptive layer is then applied.

The present invention also provides a method of detecting the presence or amount of an analyte of interest using said device.

The present invention includes, novel methods, an article for the direct selective binding, or attachment, by whatever mechanism, of the analyte of interest from serum or other bodily fluid, from solid, liquid or gaseous samples of various compositions including water, air, soil, etc., for the purpose of identification, quantitation, or separation. The present invention utilizes, in its broadest sense, a substrate coated with an intermediate layer and a receptive material that is affixed to the intermediate layer wherein an assay device is formed that has a high level of viable receptive material attached thereto. The receptive material is adapted to directly interact with the analyte of interest from the sample wherein a thin film change is produced without the use of labels as they are known in the prior art. The change in thin film or optical thickness (mass) is detected by direct optical detection methods which improve assay sensitivity.

The invention is a device or visual detection device that is formed of a substrate. This substrate is preferably formed of a light reflective material. The substrate is coated with a layer of material that functions to separate the receptive material from the unfavorable environment of the substrate and to form a favorable environment onto which the receptive material can be adhered. The receptive material can be immobilized onto the surface of this layer by a wide variety of immobilization chemistries or passive adhesion as a function of the composition of the layer. Due to the favorable environment produced by this intermediate layer the receptive material has improved reactivity, stability and interactive activity. The receptive material is selected to interact with a specific analyte of interest in the sample with a high degree of specificity.

To form the test surface, a substrate, optionally coated with an antireflective material (in the case of a visual test surface), an intermediate layer of material, and a receptive material must be selected. In an instrumented assay the type of detection system employed will determine whether the substrate should be reflective, transmissive, or absorptive. In the preferred embodiment, the instrument detection system employs an ellipsometer or an interferometer or other thin film measurement system. These instruments require the substrate to be reflective. After the appropriate substrate material has been selected, the substrate is then coated with a layer of intermediate material and with a receptive material layer. The intermediate layer provides the substrate with a microenvironment that is favorable to the viability and adherence of the selected receptive material.

The receptive material that is bound to the surface of the intermediate layer of material is characterized by the ability to interact the analyte or analytes of interest selectively. The variety of materials that can be used as a receptive material are limited only by the types of material which will interact selectively with a secondary partner. A subclass of materials which can be included in the overall class of receptive materials includes toxins, antibodies, antigens, hormone receptors, parasites, cells, haptens, metabolites, allergens, nucleic acids, nuclear materials, autoantibodies, blood proteins, cellular debris, enzymes, tissue proteins, enzyme substrates, coenzymes, and neuron transmitters, viruses, viral particles, microorganisms, metals, and various chemical species. This list incorporates only some of the many different materials that can be coated onto the intermediate layer coated substrate surface to produce a thin film assay system. Whatever the selected analyte of interest is, the receptive material is designed to interact specifically with that analyte of interest. The matrix the analyte of interest whether it is a fluid, a solid, or a gas, a bodily fluid such as mucous, saliva, urine, fecal material, tissue, bone marrow, cerebral fluid, or perspiration does not limit the utility of the present invention, as the receptive material only interacts with a specific analyte of interest in the sample and the remaining sample matrix is separated from the test surface. The test surface formed of a substrate coated with an intermediate layer of material with attached receptive material is contacted with a sample suspected to contain the analyte of interest. After the sample is contacted to the surface an instrument can be used to detect any analyte interaction. One such instrument which can be employed is the Sagax Ellipsometer (U.S. Patent #4,332,476, along with U.S. Patents 4,655,595; 4,647,207 and 4,558,012).

This invention focuses on the production of a test surface for use in thin film measurement systems, primarily optical detection systems. It describes the materials and methods of production which are utilized to produce such a test surface.

### BRIEF DESCRIPTION OF THE DRAWINGS

Figure 1 represents a prepared test surface showing the substrate, the intermediate layer, and the receptive material.
Figure 2 represents a prepared test surface reacted with analyte.
Figure 3 represents a test surface coated with antireflective material and an intermediate layer of material prior to the addition of receptive material.
Figure 4 represents a reacted portion of a prepared test surface showing those portions of the test surface reacted and unreacted with the analyte.
Figure 5 represents a dual view of reacted portion of a prepared test surface including the unreacted portion where the analyte is bound to a secondary element.
Figure 6 is a top view of Figure 5.

### DETAILED DESCRIPTION OF THE INVENTION

What follows is a description of the invention including the steps of coating the substrate 10 with the intermediate layer of material 20 and receptive material 50 to form the precoated device 60. Also shown is the method of assaying for the analyte 70 found in the sample, and the method for detecting and determining the amount of analyte 70 bound to the test surface 60. (Also shown are certain instruments that are modified and improved to give highly sensitive and accurate results.) The method of preparing the apparatus is not limited to the specific materials selected in any given example.

The use of intermediate layer material 20 between the substrate 10 and the receptive material 50 provide for a new highly sensitive assay test surface that can be reacted with a sample. The sample analyte concentration can be detected by any thin film instrument or visually. The use of the test surfaces with ellipsometers, interferometers, reflectometers, colorimeters, polarimeters. etc., permits detection of minute amounts of bound analyte 70. The addition of the intermediate layer material 20 to the test surfaces 60 improves the test surface performance. The specific advantages generated by the intermediate layer material 20 are a high density coating of viable receptive material, an increase in interactive activity, improved reactivity between the receptive material and the analyte and improved stability of the receptive material.

Numerous combinations of substrate materials, polymeric siloxanes and receptive materials can be employed to produce test surfaces for a multitude of analytes. Each analyte-specific test surface may lend itself to a number of alternate matrix-dependent assay methods. With the incorporation of anti-reflective layers between the substrate and the intermediate layer, the test surface can be tuned for interference color methods of direct visual detection. In the preferred non-instrumental embodiment of the invention, the Sagax Interference Slide U.S. Patent 4,558,012 is utilized in color interference tests. Without the need for tuned anti-reflective layers, the test surface, together with a variety of alternate optical instruments, can provide the basis for a qualitative, semi-quantitative, or quantitative assay device. In the preferred instrumented embodiment of the invention, the test surface is read on a Sagax Ellipsometer.

The following describes the parameters and necessary information for forming a test surface 60 coated with intermediate layer material 20 and receptive material 50. To simplify the discussion, an uncoated support shall be referred to as a substrate 10. A substrate 10 that is coated with intermediate layer material 20 shall be referred to as a L-coated substrate 40. A L-coated substrate 40 that has receptive material 50 attached thereto shall be referred to as R-coated substrate 60 or the test surface 60.

This invention provides for diverse substrate formats; the substrate can be a solid support, a flexible or semi-flexible support, a pellicle or a gel depending on the assay characteristics desired by the end user. Furthermore, coating the substrate material onto a support material such as glass or plastic can reduce the cost of manufacturing the assay test surface. The substrate is selected for the optical properties in virtue of which it has the appropriate reflective characteristics and for its capability of being coated with an appropriate intermediate layer 20. Visually read thin film interference effects require the substrate to be reflective and coated with an anti-reflective intermediate layer material 20. This is advantageous over the prior art because it acts as a barrier for attachment. Many of the substrates with a sufficiently reflective surface for thin film are metals which include but are not limited to: iron, stainless steel, nickel, cobalt, zinc, gold, copper, aluminum, silver, titanium, bismuth and alloys thereof. Examples of the non-metals are silicon, germanium, arsenic, carbon, silicates, aluminates, etc. These types of materials often have a surface environment which is toxic to receptive materials, especially to biological receptive materials such as antibodies, antigens, enzymes, etc.

Although the examples use a silicon wafer as a substrate, it is contemplated that a variety of substrate materials can be employed. The substrate in the examples is formed from a silicon crystal which is diamond sawed to form a wafer which is then subjected to an anisotropic etch in KOH, and then is isotropically etched to form a smoother surface. One surface of the wafer is polished producing a smooth mirror-like finish with a specularly reflecting surface. The reverse surface remains slightly irregular with ridges and valleys on the order of 200-300 nm in height producing a diffusely reflecting surface. Either side of the wafer can be employed although the specular side is the preferred for instrumented detection. Silicon wafers that are rejected by the semiconductor industry can be employed in this invention, thus reducing assay manufacturing costs. Level and type of dopant in the silicon wafer are irrelevant to this invention.

The substrate material is selected, based on whether it is sufficiently reflective and uniform to generate a signal the instrument can detect, and whether it can support the polymeric and receptive materials. It is contemplated that a transmissive substrate could be employed with various types of instrumentation. Furthermore, substrates with etched grid lines can be employed in a light scattering system. It has been noted that in some instances the substrate itself can be formed of a polymeric material. These polymeric materials must have fairly uniform surfaces that are sufficiently reflective to generate a signal in the selected instrument.

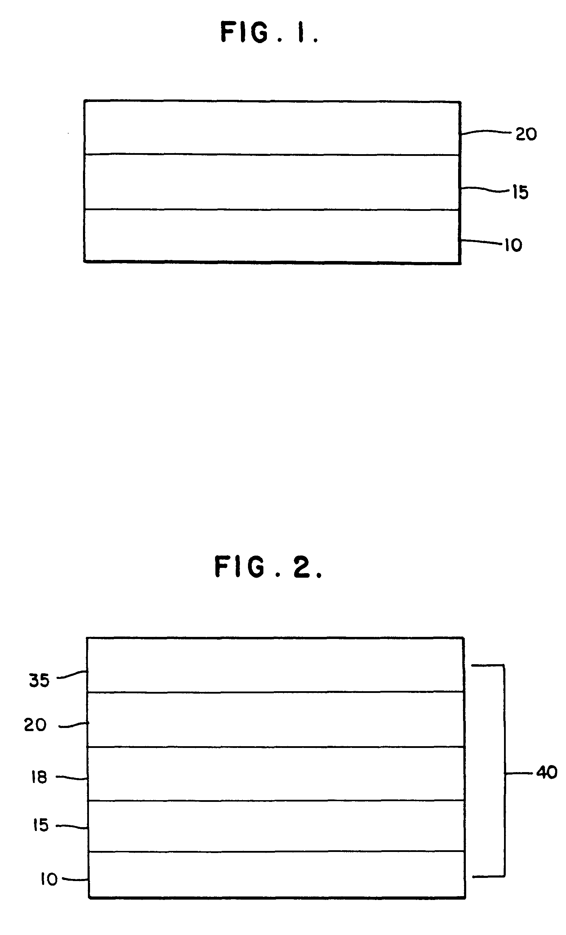

As shown in Figure 1 the substrate 10 has an upper surface 15 that is adapted to support additional material. This upper surface 15 has the optical characteristics necessary for production of a signal. The upper surface 15 should have a light reflective surface such as is produced by metals and metal alloys or silicon, etc. Adhered to the upper surface 15 is an intermediate layer of material 20. The intermediate layer material 20 is a polymeric siloxane.

The intermediate layer 20 may be produced by application of branched siloxanes. Methods of coating polymers to substrates are known to those skilled in the art of semiconductors. However, these methods are not well known in the diagnostic business. The method of polymer coating given in the examples are given as demonstration and not as limitation. It is contemplated that the polymeric materials could be attached by spin coating, aerosol spraying, dipping, etc. The method of coating can be tailored to the type of the intermediate layer material 20 employed.

Although not required, additional materials which convey a desired property may be affixed to the L-coated substrate 40. This layer could improve receptive material 50 orientation as in the use of Protein A or Protein G for orienting antibodies. Other materials which could be utilized include avidin-biotin, synthetic or recombinant Protein A/Protein G fragments or peptide or combined A/G peptide, etc.

In the preferred embodiment the intermediate material is spin coated or aerosol spray coated in a uniform manner. The various intermediate materials when coated to the substrate at thicknesses between 5 Angstroms and 500 Angstroms (thicker amounts can be employed) provide the assay test surface with the advantages previously listed. The layer is formed of polymeric siloxane that performs the following functions and has the following characteristics: creates a favorable environment for the receptive material; permits the receptive material to be densely bound in active positions by a cost-effective method; exhibits low nonspecific interacting; adheres covalently or tightly to the substrate 10; and, can be , coated to the substrate uniformly but not necessarily continuously. The intermediate layer material 20 can be placed on the substrate in patterns. Patterning can be used in a light scattering detection system, or it can be employed to generate plus/minus signals, or it can be formed as a bar code to give information, etc. Methods for patterning a substrate with polymeric material are known to those skilled in the semiconductor field.

As shown in Figures 1-3, the intermediate layer material 20 has a lower surface 18 which is attached to the upper substrate surface 15. The lower surface 18 is adapted to be compatible with the substrate 10. The polymeric material 20 also has an upper polymer surface 35 that is adapted to adhere the receptive material 50. The layer material 20 coated on the substrate 10 forms a L-coated test surface 40. The L-coated test surface 40 is usually stable and can be stored prior to coating with receptive material 50.

After the intermediate layer material is coated to the substrate forming the L-coated substrate, it may require a curing period. It has been noted that the T-polymer siloxane works best when it is given a period of time in which to cure prior to application of the chosen receptive material. The receptive material is one partner in a interactive species. As previously stated, interactive species are such things as: two chemical functional groups, antibodies-antigens, hormone-receptors, toxin-receptors, enzyme-substrate, DNA-DNA, virus-receptors, RNA-RNA, DNA-RNA, etc.

The immobilization chemistry for attaching the receptive material to the layer material is selected based on the properties of both the L-coated substrate and the receptive material. The receptive material can be covalently or passively attached to the intermediate layer material. When the intermediate layer material is specifically adapted for covalent attachment then one step in the immobilization is to activate the intermediate layer material by activation chemistry. A variety of activation and linking procedures can be employed. Usually, however, it is sufficient to passively adsorb the receptive material to the L-coated test surface thus avoiding the time and expense of immobilization chemistry procedures. Regardless of whether the receptive material is passively or covalently attached to the intermediate layer material; the thickness of the receptive material is usually less than the thickness of the intermediate layer material.

The receptive material's ability to interact with a partner (i.e., its interacting activity) is usually increased when the receptive material is attached to a L-coated substrate instead of to the substrate itself. An increase in the dynamic range is generated by having more receptive material binding sites on the test surface.

In some assay test surfaces, after the receptive material is bound, the R-coated test surface is complete. However, it may be desirable to perform a blocking. procedure. The blocking procedure can serve two functions: increase the R-coated test surface stability; and decrease nonspecific interaction. The second characteristic is the primary reason for blocking the R-coated test surface. Various blockers such as casein, porcine or bovine or human serum albumin, milk, degraded gelatin, and etc. as known to those skilled in the art can be used to prevent nonspecific interacting. The blocker is selected based on the receptive material and sample matrix employed, along with the various other factors usually addressed when selecting blocking agents.

After the R-coated test surface is blocked, it can be utilized in an analyte detection test kit. The test kit includes the R-coated test surface, calibrators, positive and negative controls (for a quantitative kit) any necessary instrument calibration instructions, and (optionally) a sample preparation and/or diluting solution. The protocol to run most thin film assays is much simpler than that of conventional solid phase assays such as RIA or EIA. The sample in whatever matrix; blood, urine, plasma, serum, aqueous solution, cerebral fluid, spinal fluid, mucous, etc., is prepared (if necessary) for contact with the test surface. This preparation step can be such procedures as cell lysing, addition of a secondary reagent, addition of sample diluent, and etc. Prepared or unprepared sample is then contacted with the test surface.

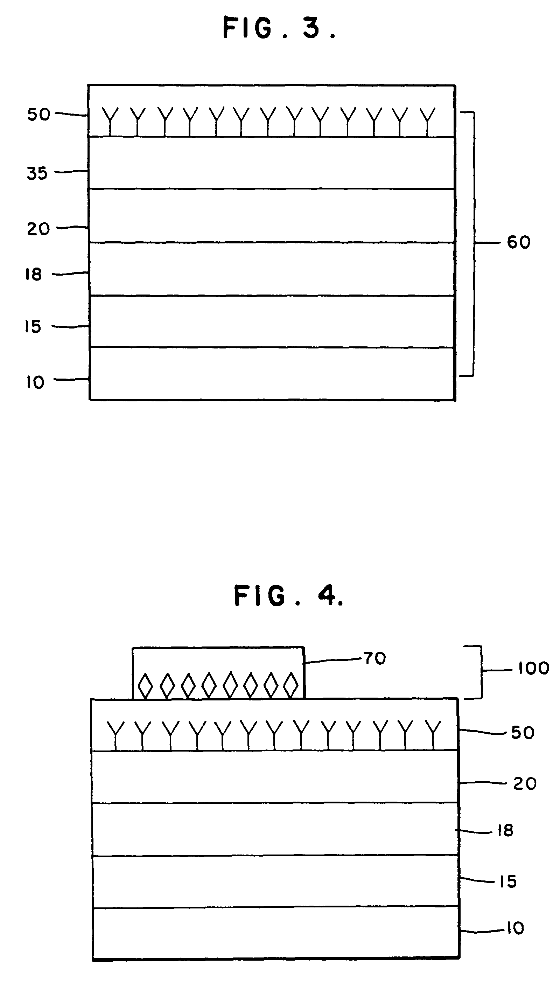

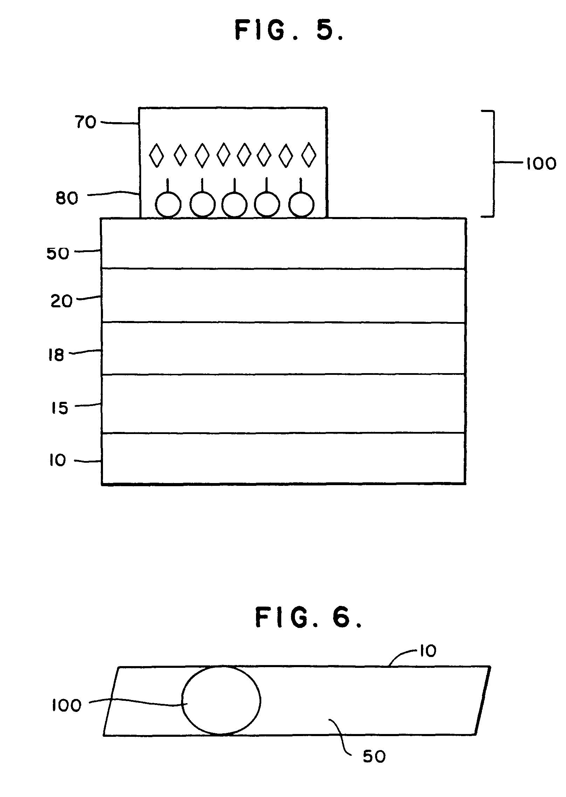

The size of the receptive material 50 and the analyte 70 in Figures 1-5 is not to scale. These two elements are enlarged to facilitate this discussion. In actual size, the thickness of the receptive material and the analyte material is usually thinner than the coating 20. The portion of the test surface 60 that is reacted with.the analyte 70 is referred to as the foreground 100, the portion of the test surface that is not contacted with the sample is referred to as the background. Figure 2 depicts only the foreground 100. The receptive material 50 that is secured to the test surface is one portion of the interactive species; the other portion of the interactive species, the analyte 70, is suspected to be in the sample as shown in Figure 4. The sample is contacted with the R-coated test surface 60 and the analyte 70 interacts to the active sites on the receptive material 50. The interaction causes a change in the mass on the surface of the R-coated test surface 60. This optical mass change is detected visually by the light interference effect or is measured by an instrument. Amplification techniques can be employed to enhance the mass change on the surface. In Figure 5, a reacted portion of a test surface is pictured. The analyte 70 is bound to a secondary reagent 80. This increases the optical mass change on the test surface 60. The test surface 60 is formed of substrate 10, with antireflective material attached thereto this is necessary only for visual detection (not shown in Figure 5). The intermediate layer material 20 is attached to the substrate 10 forming the L-coated test surface 40. The L-coated test surface 40 has a passively adsorbed or covalently attached layer of receptive material 50 forming the R-coated test surface 60. The portion of the test surface 60 depicted in Figure 5 is the foreground 100 or reacted portion. The receptive material 50 has bound the analyte of interest 70. When the sample solution was prepared the analyte 70 was combined with secondary reagent 80. Thus a greater mass change is produced on the test surface 60 by the analyte 70 and the secondary reagent 80. The amplifying material used must not adversely affect interaction between the receptive material 50 and the analyte 70 and must increase the mass change on the surface sufficiently to generate a detectable signal.

After any necessary preparation, the sample is contacted with the surface of the test surface and given a short incubation period to permit the interaction of the analyte to the receptive material on the test surface. The sample is rinsed to remove any unbound particles and dried (optionally) under a stream of gas such as pressurized air or nitrogen. The rinsing and drying steps can be performed manually, or by the test surface device holder or by a part of the instrument adapted to perform these functions.

### EXAMPLE 1

### PRODUCTION OF L-COATED SUBSTRATES

The designations given here for various intermediate layer materials will be used throughout. Intermediate Layer Materials:
#1: Comparative PEI - (Trimethoxysilylpropyl) polyethyleneimine
#2: PEI/DMDCS - Above + DimethylDichlorosilane
#3: Comparative Polystyrene
#4: T-Polymer - Aminoalkyl T-structure branch point polydimethyl siloxane
#5: DMDPS - Dimethyldiphenyl siloxane copolymer
#6: Mercapto - Mercaptopropylmethyldimethyl-siloxane copolymer
#7: Comparative BAS - N-(2-Aminoethyl-3-aminopropyl)-trimethoxysilane
#8: Comparative PBD - Triethoxysilyl modified polybutadiene
#9: PAPDS - (methylphenyl) methyldodecylmethyl-aminopropyl-methyl siloxane

### Application of Intermediate Layer Material to Substrate:

### #1: PEI (Petrarch; Bristol, PA)

A 1:500 dilution of stock silane in methanol. A 300 microliter sample of this solution was placed on a 100mm virgin test silicon wafer by micropipette, although automated aerosol or spray delivery systems are known to those skilled in the art, while the wafer was spinning at 7,000 rpm on a photoresist spin-coater. This spin-coater, utilized extensively in the semi-conductor industry, produces very uniform, conformal films. It can rapidly process a large number of substrates and is readily automated. While spin coating is detailed here, it is not the intention to limit this invention to this type of L-coated substrate production. Alternate solvent based depositions (solution or vacuum) could be easily designed by those skilled in the art. Also, while the classes of materials described here provide the protection of the receptive material from the substrate, it is not the intention of this invention to be limited to the specific materials listed except as defined in the claims. Rather it is the intention to describe the production requirements for the design of a test surface to be utilized in a thin film detection system. PEI coated substrates were cured at 100° C under 0.1 mm Hg for 60 minutes. A final intermediate layer of 80 Å as measured by conventional ellipsometry is generally preferred but other thicknesses have been utilized.

### #2: PEI/DMDCS; DMDCS (Sigma Chemical Co., St. Louis, MO)

A PEI coated substrate may be further processed by treatment with DMDCS. This creates branch points along the linear PEI chain and causes the surface to perform more as a T-polymer. A 100 milliliter stock of 2% DMDCS is prepared in 1,1,1-trichloromethane (v/v). The PEI coated substrate is submerged in the solution for 60 minutes at 25° C. The substrate is removed from the DMDCS coating solution and rinsed with 95% ethanol and finally dried under a stream of nitrogen. A final intermediate layer of 200 Å as measured by conventional ellipsometry is generally preferred, however other thicknesses are possible.

### #3: Polystyrene (Beckton Dickinson, Oxnard, CA)

Approximately 0.05g of a polystyrene falcon petri dish was dissolved in 2 milliliters of toluene. The solution was applied by the spin coating technique described above. Substrates were cured for 60 minutes at 25° C prior to utilization. A final intermediate layer of 200 Å is generally preferred, however other thicknesses are possible.

### #5: T-Polymer-(Petrarch, Bristol, PA)

A 1:300 (v/v) dilution of the T-polymer was prepared in 2-methyl-2 butanol. The intermediate layer was applied to the substrate by the spin coating method. L-coated substrate was cured for 120 minutes at 140° C prior to use. A final intermediate layer of 160 Å is generally preferred.

### #5: DMDPS (Petrarch)

A 1:100 (v/v) stock solution of the siloxane in toluene was prepared and applied utilizing the spin coating technique and curing protocol described for the T-polymer. A preferred final thickness is 200 Å.

### #6: Mercapto (Petrarch)

A 1:300 (v/v) stock solution of the siloxane was prepared in toluene. The coating and curing protocol were as described for PEI. A preferred final thickness is 200 Å.

### #7: BAS (Petrarch)

A 1:100 (v/v) solution of silane was prepared in toluene. A 200 microliter sample was used in the spin coating protocol. The wafer is cured for 2 hours under 0.1mm Hg at 140° C. A preferred final thickness is 30 Å.

### #8: PBD (Petrarch)

A 27.5 microliter volume of the stock silane was mixed with 3275 microliters of toluene. The spin coating volume was 300 microliters of this mixture and wafers were cured for 60 minutes at 120° C. A preferred final thickness is 100 Å.

### #9: PAPDS (Petrarch)

A spin coating volume of 200 microliters of 1:100 (v/v) of siloxane in toluene was used and wafers were cured for 120 minutes at 100° C prior to use. A preferred final thickness is 200 Å.

Concentrations, volumes, weights, spin coating speed, buffers, incubation time and conditions, and all other reagents or processes described throughout these examples are intended to describe preferred embodiments only. It is not the intention for this invention to be limited to these precise embodiments.

### EXAMPLE 2

### COMPARISON OF INTERMEDIATE LAYER MATERIALS UTILITY IN PRODUCTION OF A DENSE, REACTIVE RECEPTIVE LAYER; MEASURING ANTIGEN CAPTURE

A system was designed for the analysis of intermediate layer material efficiency in attaching an antibody as receptive material to the silicon substrate. Achieving a dense, reactive layer of antibody has been demonstrated in the prior art to be much more difficult than other types of receptive materials, due to more stringent orientation requirements. An ELISA system (ie, a test system not in accordance with the thin film assay system of the present invention.) was designed for this evaluation in which a monoclonal antihorseradish peroxidase (HRP) was bound to the L-coated substrate as a test receptive material, then varying levels of horseradish peroxidase (HRP) were placed on the surface in the form of a standard curve. Microtiter wells were antibody coated under the same conditions as the L-coated substrates. L-coated substrates or microtiter wells were antibody coated with a 0.05 PBS, pH=7.4 containing 20mg/ml of the monoclonal anti-HRP (Sigma Chemical Co., St. Louis, MO) for 146 hours at 25° C. The L-coated substrates were submerged in the coating solution. The peroxidase (Sigma Chemical Co., St. Louis, MO) concentrations were allowed to react with the R-coated substrate or the microtiter wells for 30 minutes at 37°C and then unbound peroxidase was removed by rinsing 'with deionized water. The TMB (Kirkegaard and Perry) substrate was then added to the reacted R-coated substrate or microtiter well and allowed to react for 2 minutes at 25° for color development. Fluid from each spot on the test surface was transferred to an uncoated microtiter well containing stopping reagent and the optical density at 450 nm recorded. Stoppong reagent was added directly to the microtiter wells of the comparison plate and it was similarly read.

The results of this study are presented in the following table. Surfaces were evaluated in term of sensitivity (resolution of low concentrations relative to the negative control) and dynamic range. For control purposes each L-coated substrate was also coated with rabbit IgG and then evaluated in the peroxidase assay. Insignificant interaction of peroxidase with all rabbit IgG coated L-coated substrates was observed. The raw silicon substrate has also been examined under similar conditions and was found to exhibit very little active receptive material binding to the surface. Data was reported as optical density measured at 450 nm.

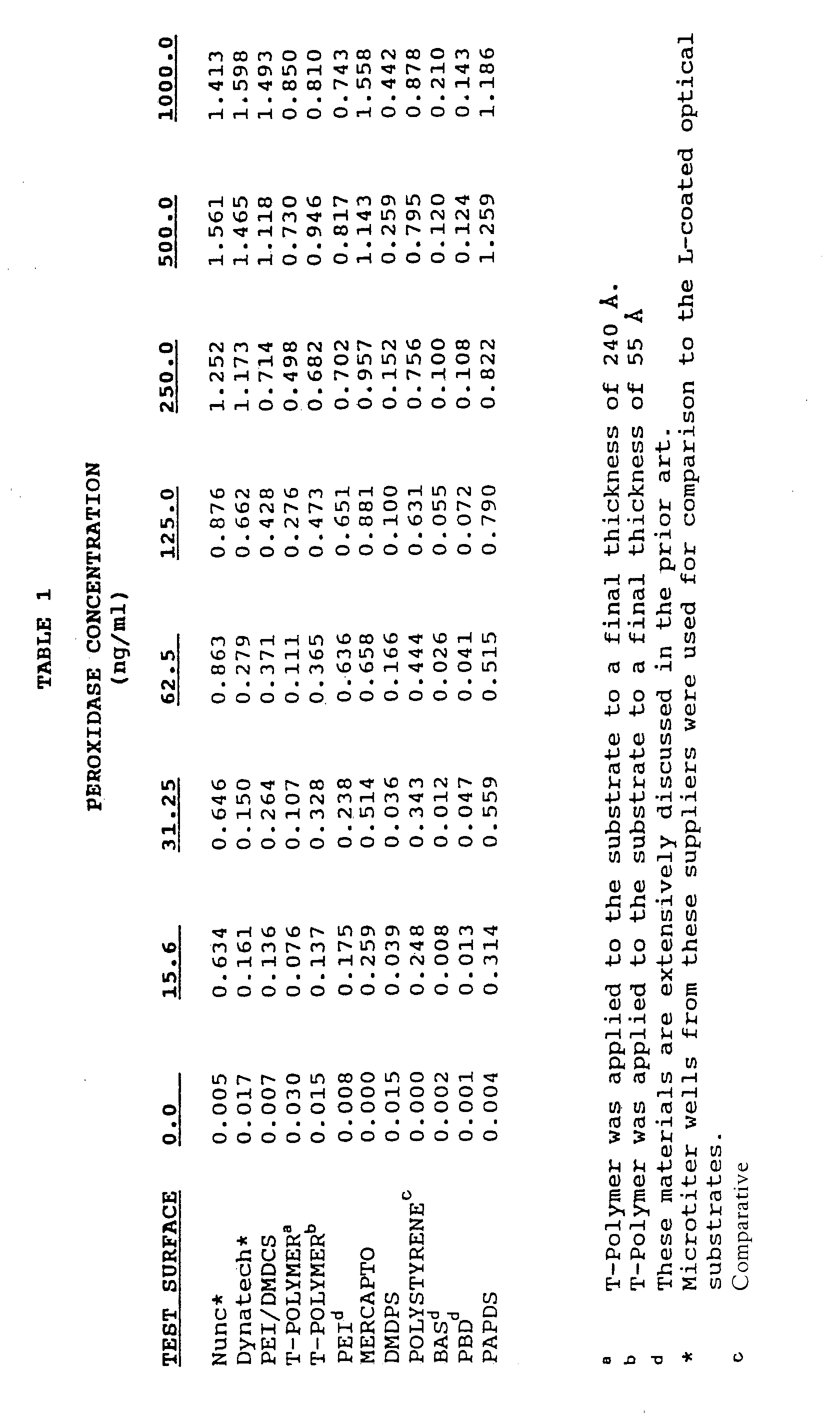

This study clearly demonstrates the utility of a siloxane as an intermediate layer on a thin film substrate relative to the prior art techniques of treating such substrates with PEI or BAS. There is also variability in the utility of the individual siloxanes, suggesting the functional groups of the siloxane may influence the reactivity of the receptive material. This study was also designed to demonstrate the utility of these intermediate materials in the constructions of a test surface where antibody is the receptive material and antigen is captured.

### EXAMPLE 3

### COMPARISON OF INTERMEDIATE LAYER MATERIALS UTILITY IN PRODUCTION OF A DENSE, REACTIVE RECEPTIVE LAYER; MEASURING ANTIBODY CAPTURE

For this analysis, varying L-coated substrates were coated by immersion in a solution of 20mg/ml of rabbit IgG (Sigma Chemical Co., St. Louis, MO) in 0.05 M PBS, pH=7.4 for 16 hours at 25° C. Different levels of a HRP labeled goat anti-(whole molecule) rabbit IgG antibody (Sigma Chemical Co., St. Louis, MO) were allowed to incubate with the R-coated substrate for 15 minutes at 37° C. Unbound material was removed by rinsing with deionized water. The TMB substrate solution was applied to the surface and allowed to react for 2 minutes at 25° C, and then the solution transferred to an uncoated microtiter well containing stopping solution. The optical density of these samples was measured at 450 nm. Data given below is in terms of Optical Density at 450 nm.

This test system is not in accordance with the thin film assay system of the present invention.

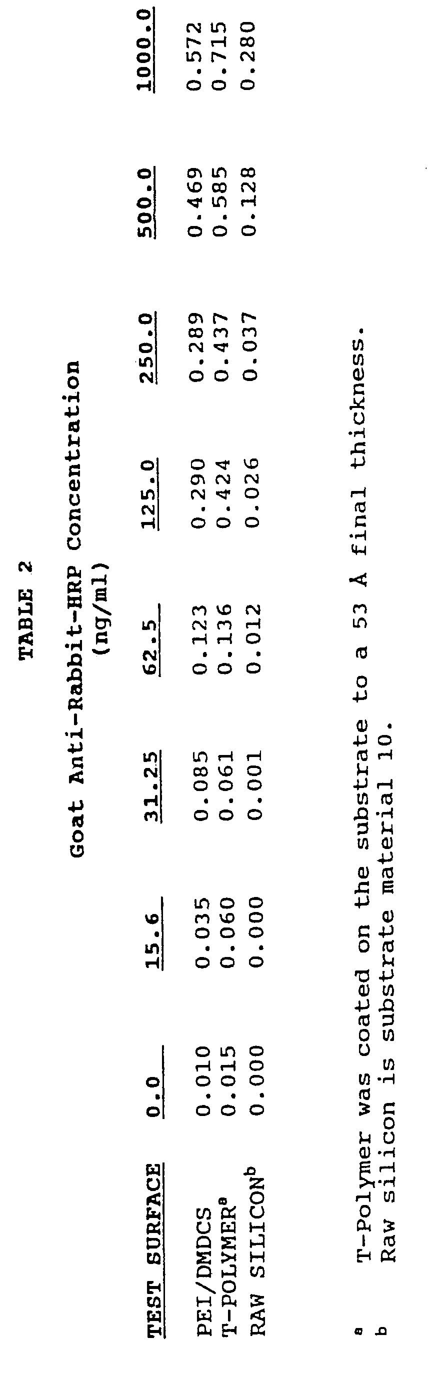

In this study the various test surfaces were used to demonstrate the utility of the system in the detection of an antibody capture assay. The substrate without the addition of an intermediate layer demonstrated very little available or reactive receptive material, supporting the need for the use of an intermediate layer of material.

### EXAMPLE 4

### INTERMEDIATE MATERIAL EVALUATION IN A VISUAL DETECTION ASSAY FOR STREPTOCOCCAL GROUP A ANTIGEN

A silicon substrate was prepared by processing diamond sawed wafers from a monocrystalline silicon ingot in a series of steps known to those skilled in the art as lapping. Sawed wafers were lapped with an abrasive material, etched to a more uniform surface profile with acid or caustic solutions, then further lapped to a progressively finer level of surface roughness. This is not as common practice among those skilled in the art where the final surface desired is highly polished planar surface. However, for this application an abrasive preparation of 12-21 micron aluminum oxide particles with a mean size of 15 microns was used to produce a diffusely reflective substrate. Reclaimed silicon wafers lapped as described are also useful at a significant cost advantage. For this particular study, the substrate prepared as described above was coated with silicon nitride to a final thickness of 550 Å. While this is the combination of materials described, any anti-reflective material at varying thicknesses may be used within this invention. The Interference Slide substrate was then treated with a number of the intermediate layer materials as described in Example 1 to produce L-coated substrate.

These L-coated substrates were coated in solution with 20 mg/ml of a rabbit anti-Streptococcus Group A (Strep A) antibody (Medix Biotech, Foster City, CA) in 0.1M HEPES, pH=6.0 for 60 minutes at 25° C. R-coated substrates were reacted by placing a 10 microliter spot of a control solution either containing or free of Strep A antigen on the R-coated substrate and incubating for 2 minutes at room temperature. Test surfaces were then rinsed with deionized water and dried under a stream of nitrogen.

The negative control was prepared by mixing 1 part 2M NaNO₂ with 1 part 2M acetic acid and neutralizing 0.66 N NaOH. The positive control, a commercially available buffer-based preparation of extracted antigen from cultured Strep A cells (Medix Biotech, Foster City, CA), was diluted in the extraction media prior to use. Samples were mixed 1:2 with a secondary reagent prior to application to the test surface. Results in Table 3 are reported as the highest dilution of positive control capable of being visualized as different from the negative control.

**TABLE 3**

| **Test Surface** | **Highest Detectable Dilution** |
|---|---|
| T-Polymer | 1:256 |
| BAS | No Visual Response |
| PEI | No Visual Response |
| PEI/DMDCS | 1:16 |
| DMDPS | No Visual Response |

This study was designed to demonstrate the utility of the intermediate materials in an antigen capture assay where the result is a visual signal. In this case the prior art materials, BAS and PEI, demonstrate no ability to insulate the receptive material from the substrate used in the visual assay system. The siloxanes demonstrate highly variable ability to provide the desired reactivity of receptive material, however, the best assay performance is obtained with the T-polymer siloxane.

### EXAMPLE 5

### INTERMEDIATE MATERIAL EVALUATION IN AN INSTRUMENTED ASSAY SYSTEM FOR STREPTOCOCCAL GROUP A ANTIGEN DETECTION

The silicon substrate used in this example is a polished wafer surface. Dopant levels and type have no impact on this type of optical detection scheme. The L-coated substrates were applied as in Example 1 and antibody was applied as in Example 4. The assay was conducted as described in Example 4. The positive control used contained a dilution of the Strep Group A antigen. Once dried, the reacted test surfaces were examined with the Sagax Comparison Ellipsometer and the photometric analysis of the reflected light intensity was recorded in terms of a millivolt reading.

**TABLE 4**

| **Test Surface** | **Negative Control** | **Positive Control** |
|---|---|---|
| | (mV) | (mV) |
| PEI | 36.0 | 133.0 |
| PBD | 21.2 | 37.7 |
| T-POLYMER | 15.5 | 286.4 |

This study was designed to provide an indication of the utility of the intermediate layer in an instrumented detection system and an antigen capture assay. For an instrumented assay, the goal was to obtain a test surface that provided maximum signal generation with a positive control and minimal signal, non-specific binding with a negative control. In this case the prior art material, PEI, demonstrates adequate reactivity, but introduces the highest level of non-specific binding. PBD which is also frequently used in the prior art, demonstrates no utility in this assay system. The best over-all performance is obtained with the T-polymer siloxane.

The foregoing examples serve to illustrate the efficiency and utility of this technology to detect a variety of analytes using the device consisting of a substrate, intermediate material(s), and receptive material(s) to generate a change in mass on the test surface as a measure of analyte attachment.

As an extension to the substrate formats or materials utilized in the preceding examples, it is possible to utilize a diversity of combinations of substrate formats and substrate materials which are functionally equivalent substitutes capable of having polymer material bound to their surface.

The polymeric siloxanes provide a layer(s) which can be activated to attach or adhere the receptive material, by whatever mechanism, to the apparatus. The polymeric siloxanes utilized in this invention are coated in a variety of manners. The composition of the polymeric siloxanes are selected based on receptive material and the substrate materials selected, and the method of attaching the receptive material.

The substrate, which can be any of a variety of shapes, i.e., test tubes, wafers, glass test surfaces, microwells, etc., and formats, i.e., a solid support, a flexible support, a gel, a pellicle and made of various suitable materials, i.e., glass, silicon, plastics, etc. and made of materials which are either nonspecularly or specularly reflective, transmissive, or absorptive can be treated with the aforementioned technology affording many useful approaches to the detection of the analyte. The reacted apparatus can be qualitatively or quantitatively analyzed by an ellipsometer, or any other instrument capable or directly optically detecting analyte interaction.

## Claims

1. A thin film device for detecting the presence or amount of an analyte of interest comprising:
(a) a substrate having a surface that provides optical characteristics compatible with direct optical detection of said analyte in a thin film assay;
(b) a conformal film intermediate layer said intermediate layer comprising a polymeric siloxane supported by the substrate surface; and
(c) a receptive material which is selectively interactive with said analyte affixed to the intermediate layer, said receptive material being affixed to the intermediate layer sufficiently densely that when said analyte is bound to said receptive material, the presence or amount of said analyte can be directly optically detected from a resulting change in mass on said surface.

2. The device of claim 1 wherein said substrate is silicon, glass, a metal or plastic; and wherein said substrate further comprises an anti-reflective material.

3. The device of claim 1 or 2, wherein said receptive material comprises a specific binding partner for said analyte.

4. The device of claim 3, wherein said receptor layer is formed from material selected from the group consisting of antigens, antibodies, oligonucleotides, chelators, enzymes, enzyme substrates, enzyme inhibitors, coenzymes, microorganisms, bacteria, parasites, toxins, cells, nucleic acids, nuclear materials, cellular debris, blood proteins, tissue proteins, allergens, metabolites, neuron transmitters, metals, viruses, viral particles, hormones and receptors for said materials.

5. The device of claim 1, wherein said analyte is an antibody, antigen, enzyme, hormone, enzyme substrate, enzyme inhibitor, chelator, metal or nucleic acid.

6. The device of claim 2, wherein said anti-reflective material is silicon nitride; said intermediate layer is aminoalkyl-T-structured branched siloxane;

7. The device of claim 1 or 2, wherein the analyte of interest is the Strep Group A antigen.

8. Method for producing an optical device as in claim 1, wherein a substrate is provided, said intermediate layer is applied by a spin coating technique, and said receptive layer is then applied.

9. Method for producing an optical device as in claim 1, wherein a substrate is provided, said intermediate layer is applied by a spray coating technique, and said receptive layer is then applied.

10. Method for producing an optical device as in claim 1, wherein a substrate is provided, said intermediate layer is applied by a dip coating technique, and said receptive layer is then applied.

11. The method of any one of claims 8 to 10, wherein said intermediate layer is a branched polymeric siloxane formed from the application of a combination of silanes.

12. The method of claim 11, wherein said intermediate layer is formed from the sequential application of a polymeric silane and dichlorodimethyl-silane.

13. The method of any one of claims 9 to 12, wherein said intermediate layer has a thickness between 5 and 500Å.

14. The device of claim 1 or 2, wherein said analyte is contained in a sample selected from the group consisting of a gas, mucous, saliva, urine, fecal extracts, tissue extracts, bone marrow, cerebral spinal fluid, perspiration, blood, plasma and serum.

15. The device of claim 1 or 2, wherein said device is adapted for use with a reflectance-photometer, an ellipsometer or an interferometer.

16. Method for detecting for presence or amount of an analyte of interest using the device of claim 1.

17. The method of claim 16 wherein said device is reacted with a sample potentially comprising said analyte under conditions in which said analyte binds to said receptive material, and creates an increase in mass on the surface of said device resulting in a detectable optical signal that allows for the detection of the presence or amount of said analyte.

18. The method of claim 17, wherein said detectable optical signal is measured with an instrument selected from a reflectance-photometer, an ellipsometer, and an interferometer.

## Patentansprüche

1. Dünnfilm-Element zum Nachweisen des Vorhandenseins oder der Menge eines interessierenden Analyts, umfassend:
(a) ein Substrat mit einer Oberfläche, welche optische Eigenschaften bietet, die mit dem direkten optischen Nachweis des Analyts in einem Dünnfilm-Assay kompatibel sind;
(b) eine Zwischenschicht aus einem konturgetreuen Film, welche Zwischenschicht ein polymeres Siloxan umfasst, das durch die Substratoberfläche getragen wird; und
(c) ein rezeptives Material, welches selektiv mit dem an der Zwischenschicht fixierten Analyt wechselwirkt, wobei das rezeptive Material an der Zwischenschicht ausreichend kompakt fixiert ist, um, wenn das Analyt an das rezeptive Material gebunden ist, das Vorhandensein oder die Menge des Analyts aus einer resultierenden Veränderung der Masse auf der Oberfläche optisch direkt nachweisen zu können.

2. Element nach Anspruch 1, wobei das Substrat Silicon, Glas, ein Metall oder ein Kunststoff ist; und wobei das Substrat außerdem ein reflexminderndes Material umfasst.

3. Element nach Anspruch 1 oder 2, wobei das rezeptive Material einen spezifischen Bindungspartner für das Analyt umfasst.

4. Element nach Anspruch 3, wobei die Rezeptorschicht aus einem Material gebildet wird, gewählt aus der Gruppe, bestehend aus Antigenen, Antikörpern, Oligonukleotiden, Chelatbildnern, Enzymen, Enzymsubstraten, Enzyminhibitoren, Coenzymen, Mikroorganismen, Bakterien, Parasiten, Toxinen, Zellen, Nukleinsäuren, Kernmaterialien, Zelltrümmern, Blutproteinen, Gewebeproteinen, Allergenen, Metaboliten, Neurotransmittern, Metallen, Viren, viralen Partikeln, Hormonen und Rezeptoren für diese Materialien.

5. Element nach Anspruch 1, wobei das Analyt ein Antikörper, Antigen, Enzym, Hormon, Enzymsubstrat, Enzyminhibitor, Chelatbildner, Metall oder Nukleinsäure ist.

6. Element nach Anspruch 2, wobei das reflexmindernde Material Siliciumnitrid ist; die Zwischenschicht Aminoalkyl-T-strukturiertes verzweigtes Siloxan ist.

7. Element nach Anspruch 1 oder 2, wobei das interessierende Analyt das Strep-Gruppe A-Antigen ist.

8. Verfahren zur Herstellung eines optischen Elements nach Anspruch 1, wobei ein Substrat bereitgestellt wird, auf das die Zwischenschicht mittels einer Spinbeschichtungsmethode aufgebracht wird und darauf die Rezeptorschicht aufgebracht wird.

9. Verfahren zur Herstellung eines optischen Elements nach Anspruch 1, wobei ein Substrat bereitgestellt wird, auf das die Zwischenschicht mittels einer Sprühbeschichtungsmethode aufgebracht wird und darauf die Rezeptorschicht aufgebracht wird.

10. Verfahren zur Herstellung eines optischen Elements nach Anspruch 1, wobei ein Substrat bereitgestellt wird, auf das die Zwischenschicht mittels einer Tauchbeschichtungsmethode aufgebracht wird und darauf die Rezeptorschicht aufgebracht wird.

11. Verfahren nach einem der Ansprüche 8 bis 10, wobei die Zwischenschicht ein verzweigtes polymeres Siloxan ist, die durch die Aufbringung einer Kombination von Silanen erzeugt wird.

12. Verfahren nach Anspruch 11, wobei die Zwischenschicht durch die aufeinander folgende Aufbringung von polymerem Silan und Dichlordimethylsilan erzeugt wird.

13. Verfahren nach einem der Ansprüche 9 bis 12, wobei die Zwischenschicht eine Dicke von zwischen 5 und 500 Å aufweist.

14. Element nach Anspruch 1 oder 2, wobei das Analyt in einer Probe enthalten ist, gewählt aus der Gruppe, bestehend aus Gas, Schleimhaut, Speichel, Urin, Stuhlextrakten, Gewebeextrakten, Knochenmark, Gehirn- und Rückenamrksflüssigkeit, Schweiß, Blut, Plasma und Serum.

15. Element nach Anspruch 1 oder 2, wobei das Element auf die Verwendung mit einem Reflexionsphotometer, einem Ellipsometer oder einer Interferometer angepasst ist.

16. Verfahren zum Nachweisen des Vorhandenseins oder der Menge eines interessierenden Analyts unter Verwendung des Elements nach Anspruch 1.

17. Verfahren nach Anspruch 16, wobei das Element mit einer Probe, die potentiell das Analyt umfasst, unter Bedingungen umgesetzt wird, in denen das Analyt an das rezeptive Material bindet, und einen Anstieg der Masse auf der Oberfläche des Elements schafft, was zu einem nachweisbaren optischen Signal führt, das die Detektion des Vorhandenseins oder der Menge des Analyts ermöglicht.

18. Verfahren nach Anspruch 17, wobei das nachweisbare optische Signal mit einem Instrument gemessen wird, gewählt aus einem Reflexionsphotometer, einem Ellipsometer und einem Interferometer.

## Revendications

1. Dispositif à film mince pour la détection de la présence ou d'une quantité d'un analyte d'intérêt comprenant :
(a) un substrat ayant une surface qui confère des caractéristiques optiques compatibles avec une détection optique directe dudit analyte dans un essai sur film mince ;
(b) une couche intermédiaire de film conformée, ladite couche intermédiaire comprenant un siloxane polymérique supporté par la surface de substrat ; et
(c) un matériau réceptif qui est sélectivement interactif avec ledit analyte fixé sur la couche intermédiaire, ledit matériau réceptif étant fixé sur la couche intermédiaire suffisamment densément pour que, lorsque ledit analyte est lié audit matériau réceptif, la présence ou une quantité dudit analyte puisse être directement détectée optiquement à partir d'un changement en masse résultant sur ladite surface.

2. Dispositif de la revendication 1, dans lequel ledit substrat est du silicium, du verre, un métal ou une matière plastique ; et dans lequel ledit substrat comprend en outre un matériau anti-réfléchissant.

3. Dispositif de la revendication 1 ou 2, dans lequel ledit matériau réceptif comprend un partenaire de liaison spécifique pour ledit analyte.

4. Dispositif de la revendication 3, dans lequel ladite couche réceptrice est formée à partir d'un matériau choisi dans l'ensemble consistant en antigènes, anticorps, oligonucléotides, agents chélatants, enzymes, substrats d'enzyme, inhibiteurs d'enzyme, coenzymes, microorganismes, bactéries, parasites, toxines, cellules, acides nucléiques, matériaux nucléaires, débris cellulaires, protéines du sang, protéines de tissus, allergènes, métabolites, neurotransmetteurs, métaux, virus, particules virales, hormones et récepteurs pour lesdits matériaux.

5. Dispositif de la revendication 1, dans lequel ledit analyte est un anticorps, un antigène, une enzyme, une hormone, un substrat d'enzyme, un inhibiteur d'enzyme, un agent chélatant, un métal ou un acide nucléique.

6. Dispositif de la revendication 2, dans lequel ledit matériau anti-réfléchissant est du nitrure de silicium et ladite couche intermédiaire est un siloxane ramifié à structure en T aminoalkylé.

7. Dispositif de la revendication 1 ou 2, dans lequel l'analyte d'intérêt est l'antigène Strep Groupe A.

8. Méthode de production d'un dispositif optique selon la revendication 1, dans laquelle un substrat est fourni, ladite couche intermédiaire est appliquée par une technique de dépôt par centrifugation, et ladite couche réceptive est ensuite appliquée.

9. Méthode de production d'un dispositif optique selon la revendication 1, dans laquelle un substrat est fourni, ladite couche intermédiaire est appliquée par une technique de dépôt par pulvérisation, et ladite couche réceptive est ensuite appliquée.

10. Méthode de production d'un dispositif optique selon la revendication 1, dans laquelle un substrat est fourni, ladite couche intermédiaire est appliquée par une technique de dépôt par immersion, et ladite couche réceptive est ensuite appliquée.

11. Méthode de l'une quelconque des revendications 8 à 10, dans laquelle ladite couche intermédiaire est un siloxane polymérique ramifié formé à partir de l'application d'une combinaison de silanes.

12. Méthode de la revendication 11, dans laquelle ladite couche intermédiaire est formée à partir de l'application séquentielle d'un silane polymérique et de dichlorodiméthylsilane.

13. Méthode de l'une quelconque des revendications 9 à 12, dans laquelle ladite couche intermédiaire a une épaisseur comprise entre 5 et 500 Å.

14. Dispositif de la revendication 1 ou 2, dans lequel ledit analyte est contenu dans un échantillon choisi dans l'ensemble consistant en un gaz, un mucus, de la salive, de l'urine, des extraits fécaux, des extraits tissulaires, de la moelle osseuse, du liquide céphalo-rachidien, de la sueur, du sang, du plasma et du sérum.

15. Dispositif de la revendication 1 ou 2, dans lequel ledit dispositif est adapté à une utilisation avec un réflectomètre-photomètre, un ellipsomètre ou un interféromètre.

16. Méthode de détection de la présence ou d'une quantité d'un analyte d'intérêt utilisant le dispositif de la revendication 1.

17. Méthode de la revendication 16, dans laquelle on fait réagir ledit dispositif avec un échantillon comprenant éventuellement ledit analyte dans des conditions dans lesquelles ledit analyte se lie audit matériau réceptif, et produit une augmentation en masse sur la surface dudit dispositif résultant en un signal optique détectable qui permet la détection de la présence ou d'une quantité dudit analyte.

18. Méthode de la revendication 17, dans laquelle ledit signal optique détectable est mesuré avec un instrument choisi parmi un réflectomètre-photomètre, un ellipsomètre et un interféromètre.
